# NEW EUROPEAN PATENT SPECIFICATION

(11) **EP 0 218 427 B2**
(45) Date of publication and mention of the opposition decision: **02.11.2000**
(45) Mention of the grant of the patent: 25.11.1992
(21) Application number: 86307420.9
(22) Date of filing: 26.09.1986
(51) Int. Cl.: A61F 9/00

(54) **Apparatus for ophthalmological surgery**
Vorrichtung für die ophthalmologische Chirurgie
Dispositif de chirurgie ophtalmologique

(30) Priority: 26.09.1985 US 780335; 31.07.1986 US 891169
(43) Date of publication of application: 15.04.1987
(73) Proprietor: VISX INCORPORATED, Santa Clara, CA 95051-0703 (US)
(72) Inventor: L'Espérance, Francis A., Englewood New Jersey 07631 (US)
(74) Representative: Milhench, Howard Leslie

(56) References cited:
- EP-A- 0 111 060
- EP-A- 0 151 869
- EP-A- 0 191 688
- EP-A- 0 412 789
- EP-B- 10 207 648
- EP-B- 10 224 322
- DE-A- 3 148 748
- FR-A- 2 576 780
- US-A- 3 330 182
- US-A- 3 703 176
- US-A- 3 843 235
- US-A- 4 156 124
- US-A- 4 461 294
- US-A- 4 648 400
- US-A- 4 784 115
- ZA-A- 847 841
- Binder et al:Arch Ophtalmol, vol.100, May 1982, pages 802-806, Refractive Keratoplasty
- Keates et al:Ophtalmic surgery, vol 12,n°2,Feb.1981,pages 117-122:Carbon dioxide laser beam control for corneal surgery
- Srinivasan:J.Vac:Sci.Technol. Vol.B1, 1983, pages 923-926
- Trokel et al:American Journal of Ophtalmology, vol. 96, 1983, pages 710-715: Excimer laser surgery of the cornea
- Draft J.J.Wynne et al "Medical application of excimer lasers" & Laser & Applications, Nov.1984, pages 59 to 62
- J.Tabaoda et al: "response of the corneal epithelium to KrF excimer laser pulses"
- Lasers & Applications, May 1986, pages 85to89, David Muller :"Excimer Lasers in Medicine"
- Wall Street Journal 30.01.87, pages 2 and 3
- Int:Journal on Laser Applications in Ophtalmology, vol 1, n° 1, May 1986, pages 21 to 48, J. Marshall et al:

## Description

### FIELD OF THE INVENTION

This invention generally concerns improvements in and relating to ophthalmological surgery and more particularly concerns improvements beyond the inventions disclosed in my European Patents Nos. EP-B-0151869 and EP-B-0207648.

### BACKGROUND OF THE INVENTION

The invention relates to that aspect of ophthalmological surgery which is concerned with operations upon the external surface of the cornea.

Operations of the character indicated include corneal transplants and keratotomies; such operations have traditionally required skilled manipulation of a cutting instrument. But, however keen the cutting edge, the mere entry of the edge into the surface of the cornea necessarily means a wedgelike lateral pressure against body cells displaced by the entry, on both sides of the entry. Such lateral pressure is damaging to several layers of cells on both sides of the entry, to the extent impairing the ability of the wound to heal, and resulting in the formation of scar tissue.

My European Patent No. EP-B-0151869 includes a background discussion of the effects of various available wavelengths of laser radiation in ophthalmological surgery and, in particular, surgery performed on the anterior surface of the cornea. It is explained that radiation at ultraviolet wavelengths is desirable by reason of its high photon energy. This energy is greatly effective on impact with tissue, in that molecules of tissue are decomposed on photon impact, resulting in tissue ablation by photodecomposition. Molecules at the irradiated surface are broken into smaller volatile fragments without heating the remaining substrate; the mechanism of the ablation is photochemical, i.e., the direct breaking of intra-molecular bonds. Photothermal and/or photocoagulation effects are neither characteristic of nor observable in ablations at ultraviolet wavelengths, and cell damage adjacent the photodecomposed ablation is insignificant. The order of magnitude of this ablative process, in the case of radiation exposure at ultraviolet wavelengths (in the range of about 400 nm or less), is that an energy density of 1 joule/cm² incises to a depth of 1 micron (1µ). Said original patent application discloses a technique of scanning a laser beam over the anterior surface of a cornea in such a controlled pattern as to sculpture said surface, imparting a new curvature to said surface, whereby to achieve optical correction of an optically deficient eye. But the scanner and scanner control to perform the technique are relatively complex and expensive.

In my European Patent No. EP-B-0207648 I describe a non-scanning technique of changing optical properties of the eye by ultraviolet laser radiation wherein controlled changes in laser-spot size perform ablative sculpturing of the cornea, resulting in a suitably corrected profile. The described technique involves programmed use of zoom-lens and/or various characterized masking techniques.

### STATEMENT OF THE INVENTION

It is an object of the present invention to provide an improved apparatus for surgically operating upon the outer surface of the cornea.

The invention, as hereinafter described with reference to exemplary embodiments, effectively fixes the position of an eye with respect to a non-scanning laser characterized by ultraviolet radiation at an energy level capable of achieving controlled ablative photodecompositon of the cornea, namely of the epithelium, Bowman's membrane and stroma levels of the cornea. Irradiated flux density and exposure time are so controlled as to achieve desired depth of the ablation.

As distinguished from the scanning and variable-spot procedures of my aforementioned previous applications, a sculpturing action results from interposing an optical screen, wedge, or mirror of precharacterized transmittance or reflectance in the path of laser-beam projection to the eye. More particularly, the cross-section of laser-beam projection to the eye is such as to accord with the full frontal area of desired curvature correction, e.g. 6 or 7-mm diameter, centered on the optical axis of the eye and the interposed device is characterized as to transmittance or reflectance which varies as a function of radius about the optical axis. In this circumstance, laser radiation at cornea impingement is of correspondingly characterized flux density, with correspondingly characterized ablative penetration capability, per unit exposure time. Thus, for myopia or hyperopia correction, the number of diopters of achieved curvature correction will be a function of exposure time, for cross-sectionally characterized radiation which is circumferentially uniform at any given radius; and for astigmatism correction, the number of diopters of achieved cylindrical correction, at a given prescribed angular orientation across the optical axis, will also be a function of time, but for cross-sectionally characterized radiation which is of symmetrically reducing flux density on opposite sides of the selected orientation axis.

According to a first aspect aspect of the invention there is provided sculpture apparatus for operation upon the external surface of the cornea of an eye of a patient, comprising laser means (13) for producing a circular output beam (12) along a predetermined beam path, said laser output beam preferably being in the ultraviolet portion of the electromagnetic spectrum, characterised in that a precharacterized beam splitter (14) is positioned in said beam path for dividing laser radiation into a reflected-component beam and into a transmitted component beam, the precharacterization of said beam-splitter being (a) such that in one of said component beams the flux-density distribution is a circumferentially uniform increasing function of radius about the central axis of said one component beam so that when the axis of said one component beam is aligned with the axis of an myopic eye, a myopia-correcting type of curvature change may be effected in the anterior surface of the cornea by exposure of the same to said one component beam for an appropriate time period, and (b) such that in the other of said component beams the flux-density distribution is a circumferentially uniform decreasing function of radius about the central axis of said other component beam so that when the axis of said other component beam is aligned with the axis of a hyperopic eye, a hyperopia-correcting type of curvature change may be effected in the anterior surface of the cornea by exposure of the same to said other component beam for an appropriate time period.

In accordance with a second aspect, the invention provides sculpture apparatus for operation upon the external surface of the cornea of an eye of a patient, comprising laser means (13) for producing a circular output beam (12) along a predetermined beam path, said laser output beam preferably being in the ultraviolet portion of the electromagnetic spectrum, characterized in that a precharacterized beam splitter (14) is positioned in said beam path for dividing laser radiation into a reflected-component beam and into a transmitted component beam, the precharacterization of said beam-splitter (14) being such that in the reflected-component beam or the transmitted component beam the flux-density distribution is a circumferentially uniform decreasing or increasing function of radius about the central axis of said beam so that when the axis of said beam is aligned with the axis of an eye, a curvature change of a myopia- or hyperopia- correcting type may be effected in the anterior surface of the cornea by exposure of the same to the precharacterized beam for an appropriate time period, and wherein energy-absorbing means is positioned in the path of whichever of the transmitted-component beam and the reflected-component beam is not aligned with the axis of the eye. Furthermore, in such sculpture apparatus said laser means can be arranged to produce a circular beam of cross-section sized for equality with the projected area of the cornea to be operated upon, said circular cross-section being precharacterized (a) by an inner circle of diameter which is but a fraction of the diameter of said cross-section and (b) by at least one circular annulus which is radially contiguous to said inner circle, the said precharacterization of said beam splitter being that of said inner circle, said circular cross-section being precharacterized further in the circular annulus thereof such that flux-density distribution is also a circumferentially uniform function of radius and in the same sense and between substantially the same density limits as apply to said inner circle, whereby a Fresnel-type corneal-curvature correction may be obtained in both said inner circle and in said circular annulus in the course of a single laser beam exposure to the cornea of the eye.

According to a third aspect, the invention provides sculpture apparatus for operation upon the external surface of the cornea of an eye of a patient, comprising laser means (13) for producing a circular output beam along a predetermined beam path, said laser output beam preferably being in the ultraviolet portion of the electromagnetic spectrum, characterized in that a precharacterized beam splitter (50) is positioned in said beam path for dividing laser radiation into a reflected-component beam and into a transmitted component beam, the precharacterization of said beam-splitter (50) being such that in the transmitted-component beam the flux-density distribution is symmetrical about a single diametral alignment through the center of said beam, said distribution being such that flux density reduces continuously and with symmetry on opposite lateral sides of said diametral alignment, and means (51) are provided mounting said beam-splitter (50) for selective rotation about the central axis of the transmitted-component beam so that an astigmatism type of curvature change may be effected in the anterior surface of a cornea by appropriate rotary adjustment of said beam-splitter (50) to a prescription axis for astigmatism correction and exposure of a cornea aligned with said transmitted-component beam for an appropriate time period.

In sculpture apparatus according to the present invention, the said beam-splitter can be centered on a circular plate and with an eye-contacting mount adapted to support said plate in slightly tilted position wherein the central geometric normal to the plate is at small angular offset from the central axis of the eye, or adapted to support said plate with its center on the alignment of the central axis of the eye.

In accordance with a feature of the present invention in all of its aspects the intensity of laser beam projections is limited per unit time to ablate but a fraction of a predetermined maximum penetration into the stroma region of the cornea, whereby a predetermined exposure time is ascertainable to achieve the predetermined maximum penetration into the stroma, and means are provided for controlling the time of precharacterized beam exposure to the cornea in accordance with said predetermined exposure time whereby depending upon the predetermined maximum penetration and the associated predetermined exposure time, one and the same precharacterizing means may be used to effect a preselected one of a plurality different predetermined diopter changes.

The above and other features of the present invention are set forth in the appended claims and will be well understood from consideration of the following detailed description given with reference to the accompanying drawings.

### BRIEF DESCRIPTION OF THE DRAWINGS

Fig. 1 is a schematic diagram in perspective, to show the general arrangement of operative components of the invention;
Fig. 2 is a simplified view in longitudinal section, showing an eye-retaining fixture used with the apparatus of Fig. 1;
Figs. 3, 4, 5, 6 and 7 are simplified diagrams to illustrate the nature of ablative corneal sculpture, performed with apparatus as in Fig. 1, for the case of correcting a myopia condition;
Figs. 8 and 9 are diagrams corresponding to Figs. 5 and 6, respectively, to illustrate ablative corneal sculpture, performed with apparatus as in Fig. 1, for the case of correcting a hyperopia condition;
Figs. 10 and 11 are schematic diagrams, respectively illustrating two different use configurations of the invention;
Figs. 12, 13, 14 and 15 are simplified diagrams to illustrate use of the invention to achieve Fresnel-type optically corrective contours at the anterior surface of the cornea, Fig. 14 graphically depicting myopia-correction, and Fig. 15 graphically depicting hyperopia-correction;
Fig. 16 is a view similar to Fig.2 to illustrate a further embodiment;
Fig. 17 is a plan view of one of a plurality of optically selectable beam-splitting elements usable in the embodiment of Fig. 16, the selected element being used in connection with laser-ablated correction for an astigmatic condition;
Fig. 18 is a diagram graphically depicting reflectance as a function of diametral distance at the section 18-18 of Fig. 17; and
Figs. 19 and 20 are similar diagrams graphically illustrating different special-purpose refinements of the invention.

### DETAILED DESCRIPTION OF EXEMPLARY EMBODIMENTS

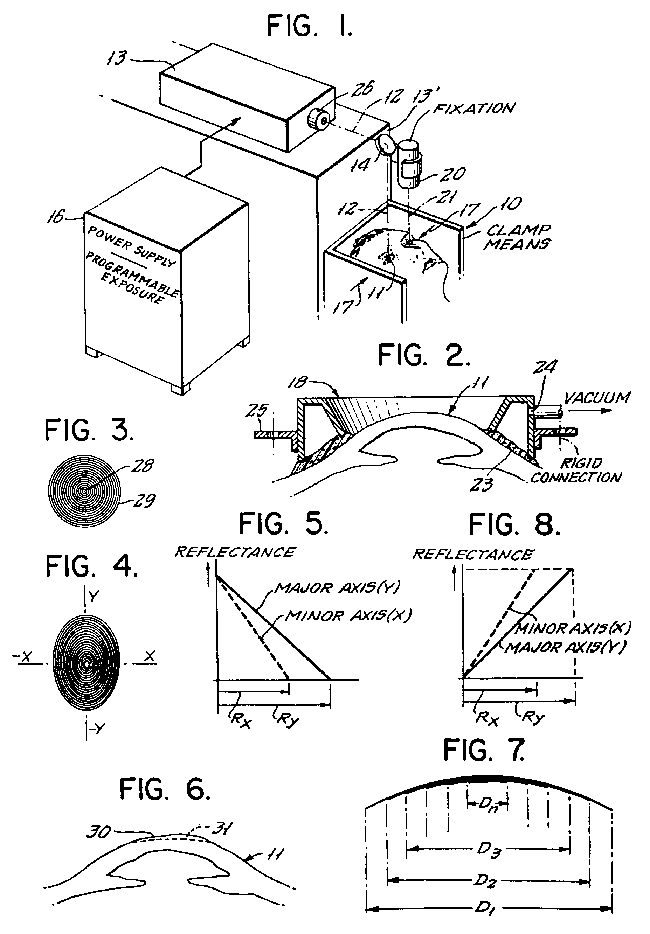

In Fig. 1, clamp means 10 is shown for fixed retention of the head of a patient (reclined, face up) such that the eye 11 to be operated upon is fixedly aligned with a downwardly folded portion 12 of the central axes 12' of beam output from a stationary laser device 13, supported by a table or other base 13'. The optical system of laser-beam projection to eye 11 includes (a) means 26 establishing the cross-section of the laser beam as a circle of 3 or 3.5-mm radius, corresponding to the corneal frontal area to be subjected to laser action, and (b) a reflector 14 of precharacterized reflectance, whereby the laser radiation incident upon the cornea is a circumferentially uniform function of radius about the central axis of the projected beam 12. A cabinet 16 is shown by legend to include a power supply for the laser, and cabinet 16 is also shown to include programmable means, which may include a microprocessor, for exposure control.

Clamp means 10 preferably includes means, symbolized at 17, to stabilize the patient's head via opposed engagements at the region of his temples, and an eye-retaining fixture 18, (Fig.2) peripherally engages eye 11 at the corneal-scleral area. Also, preferably, an optical-fixation device 20 is adjustably fixed, as to the table or base 13'. Illustratively, device 20 includes a sighting reticle and lens, whereby the eye 11' not being operated upon can view the reticle as if at infinity; the sighting alignment 21 for device 20 is parallel to the axis 12, and it will be understood that adjustable means (not shown) may provide an adjustable offset, as needed for accommodation of the patient's interpupilary distance and to adapt to the particular mounted offset of device 20 from axis 12. For an operation on the other eye 11', the eye 11 will be available for similar fixation, in conjunction with another fixation device (not shown) and associated adjustably offsetting means; alternatively, the fixation device 30 may be adjustably mounted at correct offset on the opposite side of beam 12. For purposes of operating on eye 11', clamp means 10 will have been indexed laterally with respect to laser 13 to the extent aligning axis 12 with the eye (11') then to be operated upon, thereby positioning eye 11 for use of the fixation device.

The eye-retaining fixture 18 of Fig. 2 is seen to comprise a hollow annulus, having a convergent axial-end wall 23 of air-permeable material contoured to engage and retain the eye via a scleral-corneal region. A side-port connection 24 to a vacuum pump enables retention of eye engagement to wall 23, and outward lug or flange means 25 enables rigid aligned and spaced connection of fixture 18 to laser 13 and its beam 12 via means suggested by legend in Fig. 2, such means being omitted from Fig. 1 for reasons of more simplified showing.

The laser selected for use at 13 preferably emits in the ultraviolet, namely, at wavelengths of less than substantially 400 nanometers. Such emissions for gas lasers are characteristically at 351-nm for xenon-fluoride lasers, 337-nm for nitrogen lasers, 308-nm for xenon-chloride lasers, 248-nm for krypton-fluoride lasers, 193-nm for argon fluoride lasers, and 157-nm for fluorine lasers; and within this range, frequency-doubling techniques applied to other lasers, including crystal lasers, provide further alternative sources.

One of the existing commercial excimer-laser products of Lambda Physik GmbH, Göttingen, Germany, for example their Model EMG 103 operating with argon-fluoride, is satisfactory for use as laser 13; for this product, maximum energy per pulse is 200 millijoules, with a pulse-repetition rate of 200 per second, 3 x 10⁵ shots (pulses) being available from a single charge of the involved gas, before reducing to 50 percent of specified power at this repetition rate, it being noted that full rated power is not necessarily required in use of the present invention. Pulse width is about 15 nanoseconds, and typical beam dimensions are rectangular; as shown, however, the opening in a mask 26 reduces the laser beam to a circular section.

For the situation depicted in Fig. 1, the reflecting surface of element 14 is inclined at 45 degrees to the axis of laser-beam incidence, whereby pursuant to the precharacterized nature of element 14, the laser beam is reflected on axis 12, at 90 degrees from axis 12'. with axis 12 aligned with the optical axis of eye 11. The maximum area of element 14 usable for reflecting beam 12' is thus an ellipse wherein the minor axis equals the diameter of the laser beam and the major axis is √2 times the minor axis; Fig. 3 is a simplified diagram in aid of describing the circumferentially uniform radial distribution of laser-flux density directed to eye 11, by reasons of reflection at 14, in the circumstance of precharacterized reflectance to be described in connection with Fig.5. Shading techniques are inadequate to demonstrate the precharacterized reflectance, and therefore the nature and action of such precharacterization will be described generally, first, as an optical step wedge to produce a stepped ablation profile (Fig. 7) and, then, as a smoothly progressive wedge graphically defined by Fig. 5 or Fig. 8.

Figs. 6 and 7 are illustrative of use of the invention in an optically corrective ablation of the anterior surface 30 of eye 11, wherein a myopia problem is to be solved, meaning that the curvature of surface 30 is of too-short radius to establish focus at the retina, for the case of distant objects. On the other hand, the dashed line 31 represents the ultimate curvature to which the anterior surface of the cornea should be modified to achieve a diopter-reducing corrective effect. To achieve the curve 31, the minimum desired photodecomposition is at the outer boundary 29, and the maximum is at the center 28. To produce this result, maximum laser-beam flux density characterizes ablative action at the center of the exposed area of the cornea, and minimum (zero, or substantially zero) laser-beam flux density is presented at the circumference of the exposed area. Between these radial extremes, flux density is graduated, being suggested in Fig. 3 as a succession of concentric annular bands which, for myopia reduction, will be understood to be the product of a similar succession of bands of circumferentially uniform reflectance, wherein reflectance increases as a function of decreasing radius. But since the reflector 14 must be elliptical, each of the successive bands of progressively increasing reflectance must be elliptical, as suggested by the plural ellipses of Fig. 4, having like major/minor axis ratios, wherein reflectance along the minor axis will be understood to be stepped from maximum at the center, to minimum at the extremes (-X, +X), and similarly wherein reflectance along the major axis will be understood to be stepped from maximum at the center, to minimum at the extremes (-Y, +Y).

Fig. 7 is a very much simplified diagram to represent the progressive ablative effect of a given time exposure of eye 11 to ultraviolet-laser radiation which is characterized by the described distribution of flux densities, attributable to a corresponding distribution of reflectance at 14. At the outer annulus where reflectance is least, the flux density is least and therefore little or no ablative penetration of the cornea occurs for this outer band (between diameters D₁, D₂, in Fig. 7). In the next inward annular band (between diameters D₂, D₃), an increment of reflectance accounts for an incremental advance in ablative penetration; and further such incremental advances in ablative penetration will occur in cumulative relation, as a function of decreasing radius of successive bands. The final band is a small central circle of diameter Dₙ where maximum shading thickness indicates maximum ablative penetration, due to maximum reflectance at the elliptical center of reflector 14.

The stepped progressively thicker shading of Fig. 7 (meaning correspondingly stepped increasing ablative penetration of the cornea) will be seen to define a new, larger-radius curvature for the ablated region of the cornea. Theoretically, there is a stepped character to the new profile, but for a sufficiently great number of annuli of progressively varying flux density, individual steps cease to appear discrete, and a sufficiently smooth new spherical anterior surface characterizes the cornea. This is particularly so after a post-operative period of about two days, by which time a thin epithelial layer will have spread into smooth and protective coverage of the newly characterized surface.

As indicated generally above, the stepped nature of precharacterized reflectance 14 may be replaced by a mirror surface of continuously varying reflectance, the same being graphically suggested by Fig. 5, wherein precharacterized reflectance of mirror 14 is described as being at maximum at the center, and at minimum (zero) at the periphery, whether observed along the major axis or along the minor axis, directions being shown for minor-axis increasing radial extent (R_{X}) and for major-axis increasing radial extent (R_{y}). Naturally, the ablated newly formed curvature obtained with a smoothly varying reflectance, as described for Fig. 5, will necessarily be correspondingly smooth and free of any stepped effects.

It will be seen that the amount of myopia-reducing correction achieved via precharacterized reflectance at 14 will be a function of exposure time. Thus, with a sufficient data base of diopter reduction for exposure time at given maximum flux density, it will become possible to set with precision the time required for a given diopter reduction for a given patient. For a great preponderance of cornea-curvature abnormalities, the same reflector 14 can serve to produce different degrees of curvature reduction, representing say, for relatively short exposure times, the one or two diopters of reduction needed by some patients, or, with longer exposure times, the two or more diopters of reduction needed by other patients. Also, since it may be desirable to operate upon certain patients with the caution of one increment at a time, a desired reduction of say three diopters can be achieved by a two-diopter reduction in a first visit, followed by a period of several days to permit the patient to judge and accommodate to the change, before deciding whether and to what more precise extent to make the next corrective but shorter ablative exposure, using the same apparatus and mirror 14.

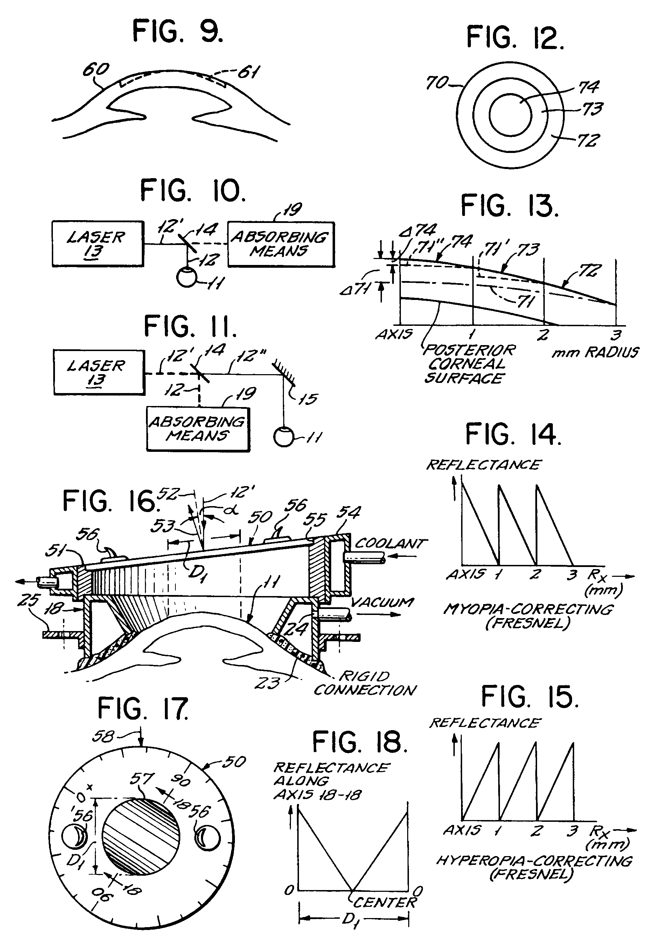

What has been said for myopia reduction applies equally for hyperopia correction, wherein reflectance at 14 must be such as to project greatest flux density of the laser beam at the outer diameter D₁ of the exposed area of the cornea, with flux density decreasing progressively to zero at the central area. This may be a stepped progression, as suggested by the multiple concentric circles of Fig. 3 (and ellipses of Fig. 4) or a continuous progression, as suggested by the curves of Fig. 8. In either event, the resulting ablated profile will be an increase in cornea curvature (i.e., shorter radius of curvature) over the exposed area, as indicated for the change in Fig. 9 from the hyperopic curvature 60 to the corrected curvature 61 (dashed line).

Described components of Fig. 1 will be recognized by reference numbers in Fig. 10, wherein the reflector 14 is shown as a beam splitter, as in the case wherein the characterized reflecting surface is applied to a suitable transparent flat substrate, as of quartz, in which case there is a transmitted beam 12'' as well as the reflected beam 12 issuing from the beam splitter. As shown, the transmitted beam 12'' is collected and dissipated by suitable means 19 which is generally designated as an absorber, but such designation will be understood to apply for the situation in which means 19 is also a means of measuring or metering dosage, in that what is transmitted will always bear a fixed proportional relation to the dosage administered via reflection along axis 12 to the eye.

Similarly, and as shown in Fig. 11, the transmitted beam on axis 12'' may be the beam used, via reflection at 15, for ablation of the cornea of eye 11, while the reflected component on axis 12 is directed to the absorbing means 19. In this event, myopia-reduction is achieved when beam splitter 14 is characterized by maximum reflectance at the maximum elliptical perimeter and by minimum (substantially zero) reflectance at the center, intervening perimeters being of progressively reducing reflectance with decreasing offset from the center. And hyperopia-reduction is achieved when reflectance is greatest at the center and minimal (substantially zero) at the maximum elliptical perimeter.

The foregoing discussion in connection with Figs. 1 to 5 presupposes a pulsed laser, exemplified by an excimer laser. But other lasers are known to emit at presently suitable energy levels and at ultraviolet wavelengths of present utility, and these other lasers will emit continuously for periods of controlled duration. For example, an organic-dye laser utilizing the proper organic dye can be made to produce laser emission in the region of 380-nm when pumped by ultraviolet laser sources such as a continuous-wave frequency-quadrupled neodymium-YAG laser operating at 266-nm; in this case, the organic-dye laser emission at 380-nm can be frequency doubled by a proper non-linear crystal such as a potassium-deuterium-phosphate (KDP) crystal or a potassium-titanium-phosphate (KTP) crystal to an emission wavelength at 190-nm. The showing of Figs. 1 to 9 will thus be understood to illustrate the further case wherein ultraviolet laser radiation on axis 12 is of continuous-wave nature, for a treatment duration predetermined by programming at 16, the timing being preset by the surgeon based on his experience, or being set as determined from a data base of accumulated experience, for diopter change as a function of exposure time.

The arrangement of Figs. 12, 13, and 14 illustrates that above-discussed principles of the invention are further applicable to corrective sculpture of the cornea to achieve a Fresnel-type distribution of the desired ultimate curvature, which can be either hyperopia-correcting or, as shown, myopia-correcting. Such an operation (i.e., Fresnel-type) would be used when, in the surgeon's considered judgment, a single smoothly developed corrected curvature would entail excessive removal of tissue at the involved region of necessarily deepest cut. To avoid too deep a cut, Figs. 12 and 13 illustrate that an ultimately reduced-curvature surface, is at 31 in Fig. 6 (dashed line 71 in Fig. 13), is achieved in annular increments within the field bounded at 70. In the outer one of these annuli (72), the curvature and depth of cut are precisely as would have applied to generate the continuous curve 71 (i.e., without Fresnel steps). But the intermediate annular area 73 effectively achieves a continuation of curve 71 with much less volume of corneal excision. Finally, the inner circular area 74 effectively completes curve 71, with minimal removal of corneal tissue.

The removal of tissue at the center is denotedd Δ74 for the Fresnel cut 74 of Figs. 12 and 13 and, comparatively, is but a small fraction of the maximum removal depth Δ71 which would have been needed to achieve the same optical correction with the smoothly developed corrected single-curvature surface 71. Fig. 14 graphically and generally depicts the precharacterized distribution of reflectance for the minor axis of the elliptical reflector, using the arrangement of Fig. 1 or Fig. 10 to achieve myopia-reducing Fresnel-type ablations of the nature described for different annuli 72, 73, 74. Within each of these annuli, greatest reflectance is at the inner dimension (R_{X}), and reflectance progressively decreases to minimum at the outer dimension (R_{X}). Thus, for a given exposure, the new curvature 71 can be achieved within outer annulus 72, with axially offset continuation of this new curvature at 71' in annulus 73; and, at further axial offset, there is further effective continuation of the new curvature at 71'', within the central circular area 74.

Fig. 15 generally depicts reflectance considerations at reflector/beam-splitter 14, for use of the Fig. 1 or Fig. 10 arrangements to achieve a hyperopia reduction. As shown, in each of the concentric annuli of a Fresnel-type ablative cut for this purpose, reflectance is minimal (substantially zero) at the inner dimension (R_{X}) and progressively increases to maximum at the outer dimension (R_{X}).

What has been said for the curves of Figs. 14 and 15 in the context of a Fig. 1 or Fig. 10 use of the invention is the exact reverse of what applies for use of the transmitted beam 12'', as in Fig. 11. Specifically, high inner-dimension reflectance, diminishing to lowest reflectance at the outer dimension of each Fresnel-type annulus (as depicted in Fig. 14) translates into greatest flux-density transmission at 12'' at the outer dimension (R_{X}), diminishing to lowest (substantially zero) flux density at the inner dimension (R_{X}) of each Fresnel-type annulus; this distribution pattern accounts for hyperopia reduction for a given exposure via axis 12''. Similarly, the reflectance characteristic depicted in Fig. 15 accounts for myopia-reduction for cornea exposures via axis 12''.

Fig. 16 illustrates an embodiment of the invention which utilizes the full circular-section beam of laser output on axis 12'' (which may have been Folded downward by a fully reflective surface, not shown), at incidence with an inclined beam-splitter 50 which is removably mounted to an adapter 51 carried by the eye-retaining fixture 18, described in connection with Fig. 2. However, in Fig. 16, the inclination angle α of normal 52 (to the surface of splitter 50) from axis 12' is purposefully small so that the characterized reflectance for the reflected component 53 may, for practical purposes, be of circular rather than elliptical nature. Fig. 3 may thus be taken with Fig. 8 in illustration of circumferentially uniform distribution of reflectance at 50 over the beam diameter D₁, where for a myopia-reducing exposure one selects a splitter 50 characterized by greatest flux density of transmitted laser radiation in the central region of the beam, decreasing to least (substantially zero) flux density at the maximum diameter D₁. And Fig. 3 may similarly be taken in conjunction with Fig. 5, in illustration of reflectance at 50 over the beam diameter D₁, where for a hyperopia-reducing exposure one selects a splitter 50 characterized by greatest flux density of transmitted laser radiation at the maximum diameter D₁, decreasing to least (substantially zero) flux density at the central region of the beam.

It will be understood that in a Fig. 16 use of the invention, the angle α should be selected such that the reflected component on axis 53 is diverted from interference with other hardware, except for interception by suitably positioned absorbing means (not shown) of the nature indicated at 19 in Figs. 10 and 11.

Fig. 16 also shows provision of an annular manifold 54 having inlet and outlet ports for accommodation of coolant flow, should heat dissipation be necessary in view of the mounting of adapter 51 in proximity to the eye 11. And to aid in on/off manipulation of selected differently characterized circular beam-splitter discs 50, with respect to nested position in the seating counterbore 55 of adapter 51, each such disc may be equipped with a pair of opposed lug fittings 56 for finger engagement outside the area (diameter D₁) of precharacterized reflectance, which also is to be understood as precharacterized transmittance, where the transmittance function is inversely related to the reflectance function.

Figs. 17 and 18 illustrate that the embodiment of Fig. 16 has further utility when the characterized circular beam-splitting area (diameter D₁) of a selectively available circular disc 50' is characterized to effect an astigmatism-reducing ablative correction via the transmitted component of beam-splitting; the characterization must be such as to pass maximum flux density on a diameter alignment across the characterized area, with progressive reduction of flux density as a function of lateral offset from the said diameter, such reductions being symmetrical on opposite sides of the said diameter alignment. In Fig. 17, shading for reflectance in the characterized region 57 of disc 50' is therefore heaviest at outermost lateral offsets from the single-diameter alignment identified with a zero-degree index marking at the edge of the disc. Other angle gradations are shown for the range to 90 degrees positive and to 90 degrees negative, being in the opposite directions away from the zero-index mark. These angles are to be road against a fixed reference mark 58 which will be understood to be inscribed in adapter 51, and it will be additionally understood that suitable keying means (not shown) or other means of angular referencing to the vertical or horizontal meridian of the eye are either provided in the assembly of adapter 51 to fixture 18 or are independently set by the surgeon so that the zero index position of disc 50' has true relation to the relevant meridian orientation. This being the case, angular indexing manipulation of disc 50' to a prescription astigmatism axis orientation with respect to reference 58 is all that is necessary for correct orientation for ablative surgery. All that then remains is to set the exposure-timing program, for ablative diopter reduction to the desired or prescribed extent.

While the invention has been described in detail for various embodiments, it will be understood that modifications may be made without departing from the scope of the invention. For example, the showings of distributed transmittance or reflectance as a linear function of radius (or effective radius) are intended as illustrative of a smooth continuum of the progression, which may be a non-linear function for certain corrective purposes.

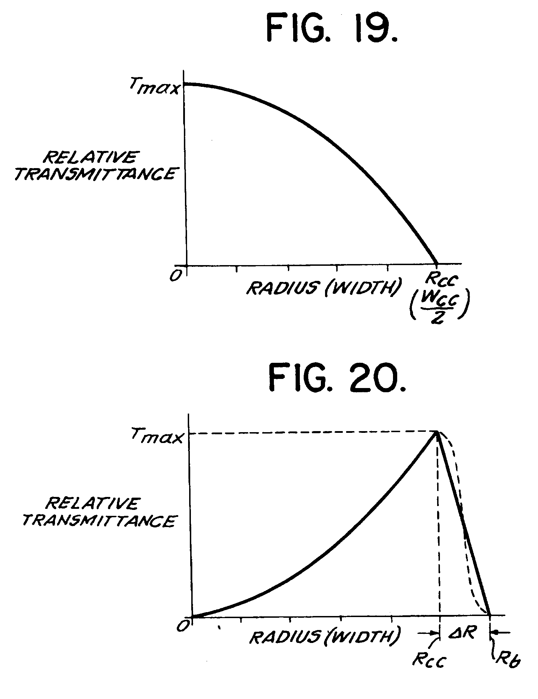

More particularly, the curves of Figs. 19 and 20 illustrate that such non-linear functions are quasiparabolic whether the exposure is myopia-correcting (Fig. 19) or hyperopia-correcting (Fig. 20). In the case of Fig. 19, maximum transmittance is at the central axis of the projected laser beam 12, so that for any diameter across a right section of said beam, the transmittance function (i.e., flux density distribution profile) is quasi-parabolic, peaking for maximum laser-beam flux density, on the central axis and reducing to zero at the maximum radius R_{cc} of the circular area in which myopia-curvature correction is to be achieved.

In the hyperopia-correcting case of Fig. 20, minimum (i.e., zero, or near-zero) transmittance is at the center of the projected laser beam 12, so that for any diameter across a ring section of said beam, the transmittance function (i.e., flux-density distribution profile) is quasi-parabolic, peaking, for maximum laser-beam flux density, at the radially outer limit R_{cc} of the circular area in which hyperopia-curvature correction is to be achieved.

It will be recalled from the hyperopia-correcting discussion in connection with Fig. 9 that deepest penetration of the stroma is at the perimeter of the area of surgery, thus leaving a relatively sharp circular edge, of depth proportional to the quantum of exposure to the laser surgery, i.e., proportional to the magnitude of diopter correction achieved. Such a sharp edge produces a problem for epithelial regrowth over the area of surgery, in that epithelial regrowth is optimum for essentially continuous surfaces, uninterrupted by sharp edges or by sharp discontinuities. To avoid such a sharp-edge development, Fig. 20 additionally shows that the projected laser beam 12 should be of sectional area larger than that over which hyperopia-curvature correction is to be achieved, thus providing for an outer profile-smoothing annulus contiguous to and surrounding the circle of curvature-correction. In Fig. 20, the incremental radius Δ R defines this annulus; and reduction in transmittance, from maximum at R_{cc} to minimum at the radius R_{b} of the projected laser beam, is shown to be linear, in the radially outward direction, and between inner and outer limits of the annulus Δ R. Generally, the radial extent Δ R of the annulus should be in the range of 5 to 15 percent of the radius R_{cc} of the circle of curvature correction, and preferably about 10 percent.

It will be understood that the indicated linear reduction in transmittance will account for minimum slope at all points within the annulus, meaning that for deepest surgical penetration of the cornea (e.g., 100 microns, for a 10-diopter correction over a 5-mm diameter circle of curvature correction), a linear characteristic is best; but for lesser penetrations such as for diopter corrections of up to 5 diopters, a non-linear relationship (as suggested by the dashed-line curve spanning Δ R in Fig. 20) enables provision (within the radial span Δ R) of continuously smooth curvature transition, from the radius R_{cc} of maximum penetration and radially outward to the untreated adjacent original profile of the cornea.

What has been said above as to minimizing and eliminating sharp-edge development for a hyperopia-correcting sculpture of the cornea also applies for the case of astigmatism-correcting sculpture when the astigmatism is hyperopia-analogous, i.e., when astigmatism-correction requires an increase in the cylindrical radius of curvature in order to reduce or eliminate the astigmatism. In such case, maximum depth of corrective sculpturing penetration of the cornea is at the laterally outer limits of the astigmatism-correcting procedure, leaving relatively sharp edges at these outer limits. These edges are avoided or materially reduced in severity if the laser beam is so precharacterized, laterally outward of these maximum-depth limits, as to reduce in approach to substantially zero flux density. If the curve of Fig. 20 is taken to show relative transmittance (flux-density distribution) laterally outward of the predetermined orientation of astigmatism to be corrected (rather than in terms of radial distribution), then the profile of Fig. 20 is seen as a half section of the transmittance distribution normal to the predetermined direction of astigmatism correction. In similar fashion, Fig. 19 illustrates such distribution for the case of astigmatism that is myopia-analogous, and parenthetical ("width") legends in both Figs. 19 and 20 can be taken as a showing of the respective astigmatism-correcting profiles.

In the foregoing discussion, fairly consistent reference has been made to reflectors 14 and 50 as beam splitters, i.e., with capability both to transmit and to reflect characterized distributions of transmittance and reflectance properties.

## Claims

1. Sculpture apparatus for operation upon the external surface of the cornea of an eye of a patient, comprising laser means (13) for producing a circular output beam (12) along a predetermined beam path, said laser output beam preferably being in the ultraviolet portion of the electromagnetic spectrum, characterised in that a precharacterized beam splitter (14) is positioned in said beam path for dividing laser radiation into a reflected-component beam and into a transmitted component beam, the precharacterization of said beam-splitter being (a) such that in one of said component beams the flux-density distribution is a circumferentially uniform increasing function of radius about the central axis of said one component beam so that when the axis of said one component beam is aligned with the axis of an myopic eye, a myopia-correcting type of curvature change may be effected in the anterior surface of the cornea by exposure of the same to said one component beam for an appropriate time period, and (b) such that in the other of said component beams the flux-density distribution is a circumferentially uniform decreasing function of radius about the central axis of said other component beam so that when the axis of said other component beam is aligned with the axis of a hyperopic eye, a hyperopia-correcting type of curvature change may be effected in the anterior surface of the cornea by exposure of the same to said other component beam for an appropriate time period.

2. Sculpture apparatus for operation upon the external surface of the cornea of an eye of a patient, comprising laser means (13) for producing a circular output beam (12) along a predetermined beam path, said laser output beam preferably being in the ultraviolet portion of the electromagnetic spectrum, characterized in that a precharacterized beam splitter (14) is positioned in said beam path for dividing laser radiation into a reflected-component beam and into a transmitted component beam, the precharacterization of said beam-splitter (14) being such that in the reflected-component beam or the transmitted component beam the flux-density distribution is a circumferentially uniform decreasing or increasing function of radius about the central axis of said beam so that when the axis of said beam is aligned with the axis of an eye, a curvature change of a myopia- or hyperopia- correcting type may be effected in the anterior surface of the cornea by exposure of the same to the precharacterized beam for an appropriate time period, and wherein energy-absorbing means (19) is positioned in the path of whichever of the transmitted-component beam and the reflected-component beam is not aligned with the axis of the eye.

3. Sculpture apparatus according to claim 2, in which said laser means (13) produces a circular beam of cross-section sized for equality with the projected area of the cornea to be operated upon, said circular cross-section being precharacterized (a) by an inner circle (Dₙ) of diameter which is but a fraction of the diameter of said cross-section and (b) by at least one circular annulus (D₁, D₂ D₃...) which is radially contiguous to said inner circle, the said precharacterization of said beam splitter (14) being that of said inner circle (Dₙ), said circular cross-section being precharacterized further in the circular annulus (D₁, D₂, D₃...) thereof such that flux-density distribution is also a circumferentially uniform function of radius and in the same sense and between substantially the same density limits as apply to said inner circle (Dₙ), whereby a Fresnel-type corneal-curvature correction may be obtained in both said inner circle (Dₙ) and in said circular annulus (D₁, D₂, D₃...) in the course of a single laser-beam exposure to the cornea of the eye.

4. Sculpture apparatus for operation upon the external surface of the cornea of an eye of a patient, comprising laser means (13) for producing a circular output beam along a predetermined beam path, said laser output beam preferably being in the ultraviolet portion of the electromagnetic spectrum, characterized in that a precharacterized beam splitter (50) is positioned in said beam path for dividing laser radiation into a reflected-component beam and into a transmitted component beam, the precharacterization of said beam-splitter (50) being such that in the transmitted-component beam the flux-density distribution is symmetrical about a single diametral alignment through the center of said beam, said distribution being such that flux density reduces continuously and with symmetry on opposite lateral sides of said diametral alignment, and means (51) are provided mounting said beam-splitter (50) for selective rotation about the central axis of the transmitted-component beam so that an astigmatism type of curvature change may be effected in the anterior surface of a cornea by appropriate rotary adjustment of said beam-splitter (50) to a prescription axis for astigmatism correction and exposure of a cornea aligned with said transmitted-component beam for an appropriate time period.

5. Sculpture apparatus according to any of claims 1 to 4 in which said beam-splitter (50) is centered on a circular plate (51), and in which an eye-contacting mount (18) is adapted to support said plate (51) in slightly tilted position wherein the central geometric normal to the plate is at small angular offset from the central axis of the eye or is adapted to support said plate with its center on the alignment of the central axis of the eye.

6. Sculpture apparatus according to claim 2 or any claim dependent thereon in which said flux-density distribution (i) is a circumferentially uniform increasing function of radius out to a maximum flux density at a first radial limit of hyperopia-corrected area, and (ii) is a circumferentially uniform decreasing function of radius, from said maximum and out to a minimum at a second radial limit of laser-beam cross-section.

7. Sculpture apparatus according to any of the preceding claims wherein the intensity of laser beam projection is limited to a predetermined level such that only a fraction of a predetermined maximum penetration into the stroma region of the cornea is achievable in a predetermined time period, and means (16) are provided for controlling the time of precharacterized beam exposure to the cornea so that one and the same precharacterizing means may be used to effect a preselected one of a plurality of different predetermined diopter changes.

8. Sculpture apparatus according to any of the preceding claims comprising means (17) to stabilize the patient's head relative to the laser beam.

9. Sculpture apparatus according to any of the preceding claims comprising an optical fixation device (20) including a sighting reticule and lens whereby the eye not being operated upon can view the reticule as if at infinity.

## Patentansprüche

1. Formgebungsvorrichtung zur Operation auf der äußeren Oberfläche der Hornhaut eines Auges eines Patienten, die zur Erzeugung eines kreisförmigen Ausgangsstrahls (12) längs eines vorbestimmten Strahlenganges eine Laservorrichtung (13) enthält, wobei der Laserausgangsstrahl vorzugsweise im ultravioletten Bereich des elektromagnetischen Spektrums liegt, **dadurch gekennzeichnet, daß** eine vorcharakterisierte Strahlspalteinrichtung (14) in dem Strahlengang angeordnet ist, um den Laserstrahl in eine Reflexionsstrahlkomponente und in eine Übertragungsstrahlkomponente zu zerlegen, wobei die Vorcharakterisierung der Strahlspalteinrichtung (a) so ist, daß in einer der Strahlkomponenten die Feldstärkeverteilung eine umfangsgleiche, steigende Funktion des Radius um die Mittelachse der einen Strahlkomponente darstellt, sodaß, wenn die Achse der einen Strahlkomponente mit der Achse eines kurzsichtigen Auges ausgerichtet ist, eine Krümmungsänderung des die Kurzsichtigkeit korrigierenden Typs in der vorderen Oberfläche der Hornhaut beim Aussetzen derselben der einen Strahlkomponente für eine geeignete Zeitperiode bewirkt werden kann und (b) so, daß in der anderen der Strahlkomponenten die Feldstärkeverteilung eine umfangsgleiche, fallende Funktion des Radius um die Mittelachse der anderen Strahlkomponente darstellt, sodaß, wenn die Achse der anderen Strahlkomponente mit der Achse eines übersichtigen Auges ausgerichtet ist, eine Krümmungsänderung des die Übersichtigkeit korrigierenden Typs in der vorderen Oberfläche der Hornhaut durch Aussetzen derselben der anderen Strahlkomponente für eine geeignete Zeitperiode bewirkt werden kann.

2. Formgebungsvorrichtung zur Operation auf der äußeren Oberfläche der Hornhaut des Auges eines Patienten, die eine Laservorrichtung (13) zur Erzeugung eines kreisförmigen Ausgangsstrahl (12) längs eines vorbestimmten Strahlenganges enthält, wobei der Laserausgangsstrahl vorzugsweise im ultravioletten Bereich des elektromagnetischen Spektrums liegt, **dadurch gekennzeichnet, daß** eine vorcharakterisierte Strahlspalteinrichtung (14) in dem Strahlengang angeordnet ist, um Laserstrahlung in eine Reflexionsstrahlkomponente und in eine Übertragungsstrahlkomponente zu zerlegen, wobei die Vorcharakterisierung der Strahlspalteinrichtung (14) so beschaffen ist, daß in der Reflexionsstrahlkomponente oder in der Übertragungsstrahlkomponente die Feldstärkeverteilung eine umfangsgleiche, fallende oder ansteigende Funktion des Radius um die Mittelachse des Strahls darstellt, sodaß, wenn die Achse des Strahls mit der Achse eines Auges ausgerichtet ist, eine Krümmungsänderung des die Kurzsichtigkeit oder die Übersichtigkeit korrigierenden Typs in der vorderen Oberfläche der Hornhaut durch Aussetzen derselben dem vorcharakterisierten Strahl für eine geeignete Zeitperiode bewirkt werden kann, und wobei ein Energie absorbierendes Mittel (19) im Strahlengang derjenigen aus der Übertragungsstrahlkomponente und der Reflexionsstrahlkomponente angeordnet ist, die nicht nach der Achse des Auges ausgerichtet ist.

3. Formgebungsvorrichtung nach Anspruch 2, in der die Laservorrichtung (13) einen kreisförmigen Strahl mit einem Querschnitt von derselben Größe wie der ausgesetzte Bereich der Hornhaut, auf dem operiert werden soll, erzeugt, in der der kreisförmige Querschnitt vorcharakterisiert ist (a) durch einen Innenkreis (Dₙ) mit einem Durchmesser, der nur einen Bruchteil des Durchmessers des Querschnittes ausmacht und (b) durch wenigstens einen Kreisring (D₁, D₂, D₃...), der radial an den Innenkreis angrenzt, die Vorcharakterisierung der Strahlspalteinrichtung (14) entspricht dem Innenkreis (Dₙ), der kreisförmige Querschnitt wird weiter durch die Kreisringe (D₁, D₂, D₃...) so vorcharakterisiert, daß die Feldstärkeverteilung ebenfalls eine umfangsgleiche Funktion des Radius und mit der gleichen Bedeutung zwischen im wesentlichen den gleichen Verteilungsgrenzen als Anwendung des Innenkreises (Dₙ) darstellt, wodurch sowohl im Innenkreis (Dₙ) als auch in den Kreisringen (D₁, D₂, D₃...) im Verlauf einer einzigen Laserstrahlbelichtung auf die Hornhaut des Auges eine dem Fresnel-Typ entsprechende Änderung der Hornhautkrümmung erreicht werden kann.

4. Formgebungsvorrichtung zur Operation auf der äußeren Oberfläche der Hornhaut eines Auges eines Patienten, die eine Laservorrichtung (13) zur Erzeugung eines kreisförmigen Ausgangsstrahls längs eines vorbestimmten Strahlenganges enthält, wobei der Laserausgangsstrahl vorzugsweise im ultravioletten Bereich des elektromagnetischen Spektrums liegt, **dadurch gekennzeichnet, daß** eine vorcharakterisierte Strahlspalteinrichtung (50) im Strahlengang angeordnet ist, um den Laserstrahl in eine Reflexionsstrahlkomponente und eine Übertragungsstrahlkomponente zu zerlegen, wobei die Vorcharakterisierung der Strahlspalteinrichtung (50) so gestaltet ist, daß die Feldstärkeverteilung in der Übertragungstrahlkomponente um eine einzige Durchmesserausrichtung durch die Mitte des Strahls symmetrisch ist, die Verteilung so beschaffen ist, daß sich die Feldstärke gleichmäßig und symmetrisch zu den gegenüberliegenden Querseiten der Durchmesserausrichtung verringert, und es sind Mittel (51) vorgesehen, um die Strahlspalteinrichtung (50) zur wahlweisen Drehung um die Mittelachse der Übertragungstrahlkomponente zu halten, sodaß eine dem Astigmatismus-Typ entsprechende Krümmungsänderung auf der vorderen Oberfläche der Hornhaut durch geeignete Dreheinstellung der Strahlspalteinrichtung (50) auf eine vorgeschriebene Achse für Astigmatismus-Korrektur und Belichtung einer zur Übertragungsstrahlkomponente ausgerichteten Hornhaut zu einer geeigneten Zeitperiode bewirkt werden kann.

5. Formgebungsvorrichtung nach einem der Ansprüche 1 bis 4, in der die Strahlspalteinrichtung (50) auf einer Kreisplatte (51) mittig angeordnet ist, und in der eine Augenkontakthalterung (18) angepaßt ist, um die Platte (51) in leicht geneigter Stellung zu unterstützen, in der die geometrische Mittelnormale zur Platte leicht winkelversetzt zur Mittelachse des Auges ist oder angepaßt ist, um die Platte in ihrer Mitte ausgerichtet mit der Mittelachse des Auges zu halten.

6. Formgebungsvorrichtung nach Anspruch 2 oder einen davon abhängigen Anspruch, in der die Feldstärkeverteilung (i) eine umfangsgleiche, ansteigende Funktion des Radius auf eine maximale Feldstärke bei einer ersten radialen Begrenzung des korrigierten Übersichtigkeitsbereiches und (ii) eine umfangsgleiche, fallende Funktion des Radius von dem Maximum auf ein Minimum bei einer zweiten radialen Begrenzung des Laserstrahlquerschnittes darstellen.

7. Formgebungsvorrichtung nach einem der vorangehenden Ansprüche in der die Intensität der Laserbestrahlung so auf ein vorbestimmtes Niveau begrenzt ist, daß nur ein Bruchteil der vorbestimmten maximalen Durchdringung in den Stromabereich der Hornhaut in einer vorbestimmten Zeitperiode erreichbar ist, und in der zur Kontrolle der Dauer der vorcharakterisierten Laserstrahlbelichtung auf die Hornhaut Einrichtungen (16) vorgesehen sind, sodaß ein und dasselbe Vorcharakterisierungsmittel angewendet werden kann, um eine aus einer Vielzahl von verschiedenen vorbestimmten Dioptrieänderungen ausgewählte zu erzielen.

8. Formgebungsvorrichtung nach einem der vorangehenden Ansprüche, mit einer Einrichtung (17) zum Stabilisieren des Kopfs des Patienten relativ zum Laserstrahl.

9. Formgebungsvorrichtung nach einem der vorangehenden Ansprüche mit einer optischen Feststellvorrichtung (20) mit einem Beobachtungsfadenkreuz und einer Linse, durch die das Auge, auf dem nicht operiert wird, das Fadenkreuz wie im Unendlichen sehen kann.

## Revendications

1. Appareil de sculpture pour opération sur la surface externe de la cornée d'un oeil d'un patient, comprenant des moyens laser (13) pour produire un faisceau de sortie circulaire (12) le long d'un trajet de faisceau prédéterminé, ledit faisceau laser de sortie étant de préférence pris dans la partie ultraviolette du spectre électromagnétique, caractérisé en ce qu'un fractionneur de faisceau précaractérisé (14) est positionné dans ledit trajet de faisceau pour diviser la radiation laser en une composante de faisceau réfléchie et une composante de faisceau transmise, la précaractérisation dudit fractionneur de faisceau étant (a) telle que dans l'une desdites composantes de faisceau, la distribution de densité de flux est une fonction croissante, circulairement uniforme, du rayon autour de l'axe central de ladite composante de faisceau, de telle façon que lorsque l'axe de ladite composante de faisceau est aligné avec l'axe d'un oeil myope, une modification de courbure du type correcteur de myopie peut être exécutée dans la surface antérieure de la cornée par son exposition à ladite composante de faisceau pendant une période de temps appropriée, et (b) telle que dans l'autre desdites composantes de faisceau, la distribution de densité de flux est une fonction décroissante, circonférentiellement uniforme, du rayon autour de l'axe central de ladite autre composante de faisceau de telle façon que lorsque l'axe de ladite autre composante de faisceau est aligné avec l'axe d'un oeil hypermétrope, une modification de courbure du type correcteur d'hypermétropie peut être exécutée dans la surface antérieure de la cornée par son exposition à ladite autre composante de faisceau pendant une période de temps appropriée.

2. Appareil de sculpture pour opération sur la surface externe de la cornée d'un oeil d'un patient, comprenant des moyens laser (13) pour produire un faisceau de sortie circulaire (12) le long d'un trajet de faisceau prédéterminé, ledit faisceau laser de sortie étant de préférence pris dans la partie ultraviolette du spectre électromagnétique, caractérisé en ce qu'un fractionneur de faisceau précaractérisé (14) est positionné dans ledit trajet de faisceau pour diviser la radiation laser en une composante de faisceau réfléchie et une composante de faisceau transmise, la précaractérisation dudit fractionneur de faisceau (14) étant telle que dans la composante de faisceau réfléchie ou dans la composante de faisceau transmise, la distribution de densité de flux est une fonction croissante ou décroissante, circonférentiellement uniforme, du rayon autour de l'axe central dudit faisceau, de telle façon que lorsque l'axe dudit faisceau est aligné avec l'axe d'un oeil, une modification de courbure d'un type correcteur de myopie ou d'hypermétropie peut être exécutée dans la surface antérieure de la cornée par son exposition au faisceau précaractérisé pendant une période de temps appropriée, et dans lequel un moyen d'absorption d'énergie (19) est positionné dans le trajet de n'importe laquelle parmi la composante de faisceau transmise et la composante de faisceau réfléchie, qui n'est pas alignée avec l'axe de l'oeil.

3. Appareil de sculpture selon la revendication 2, dans lequel lesdits moyens laser (13) produisent un faisceau circulaire de section transversale dimensionnée pour être égale à la surface projetée de la cornée qui doit être opérée, ladite section transversale circulaire étant précaractérisée (a) par un cercle intérieur (Dₙ) d'un diamètre qui n'est qu'une fraction du diamètre de ladite section transversale et (b) par au moins un anneau circulaire (D₁, D₂, D₃ ...) qui est contigu radialement audit cercle intérieur, ladite précaractérisation dudit fractionneur de faisceau (14) étant celle dudit cercle (Dₙ), ladite section transversale circulaire étant en outre précaractérisée dans son anneau circulaire (D₁, D₂, n₃ ...) de telle façon que la distribution de la densité de flux est également une fonction circonférentiellement uniforme du rayon et dans le même sens et entre sensiblement les mêmes limites de densité que celles qui s'appliquent audit cercle intérieur (Dₙ), ce par quoi une correction de courbure cornéenne du type Fresnel peut être obtenue tant dans ledit cercle intérieur (Dₙ) que dans ledit anneau circulaire (D₁, D₂, D₃ ...) au cours d'une exposition unique au rayon laser de la cornée de l'oeil.

4. Appareil de sculpture pour opération sur la surface externe de la cornée d'un oeil d'un patient, comprenant des moyens laser (13) pour produire un faisceau de sortie circulaire le long d'un trajet de faisceau prédéterminé, ledit faisceau laser de sortie étant de préférence pris dans la partie ultraviolette du spectre électromagnétique, caractérisé en ce qu'un fractionneur de faisceau précaractérisé (50) est positionné dans ledit trajet de faisceau pour diviser la radiation laser en une composante de faisceau réfléchie et une composante de faisceau transmise, la précaractérisation dudit fractionneur de faisceau (50) étant telle que dans la composante de faisceau transmise, la distribution de densité de flux est symétrique par rapport à un alignement diamétral unique passant par le centre dudit faisceau, ladite distribution étant telle que la densité de fluide diminue continuellement et symétriquement sur les côtés opposés dudit alignement diamétral, et des moyens (51) sont prévus pour monter ledit fractionneur de faisceau (50) en vue d'une rotation sélective autour de l'axe central de ladite composante de faisceau transmise de telle façon qu'une modification de courbure du type astigmatisme peut être effectuée dans la surface antérieure d'une cornée par un réglage angulaire approprié dudit fractionneur de faisceau (50) sur un axe de prescription pour correction de l'astigmatisme et exposition d'une cornée alignée avec ladite composante de faisceau transmise pendant une période de temps appropriée.

5. Appareil de sculpture selon l'une quelconque des revendications 1 à 4, dans lequel ledit fractionneur de faisceau (50) est centré sur une plaque circulaire (51), et dans lequel une monture de contact avec l'oeil (18) est adaptée pour supporter ladite plaque (51) en position légèrement inclinée dans laquelle la normale géométrique centrale à la plaque est en léger décalage angulaire par rapport à l'axe central de l'oeil, ou est adaptée pour supporter ladite plaque avec son centre sur l'alignement de l'axe central de l'oeil.

6. Appareil de sculpture selon la revendication 2 ou l'une quelconque des revendications qui en dépendent, dans lequel ladite distribution de densité de flux (i) est une fonction croissante, circonférentiellement uniforme, du rayon vers une densité de flux maximale à une première limite radiale de surface ayant subi une correction d'hypermétropie et (ii) est une fonction décroissante, circonférentiellement uniforme, du rayon, depuis ledit maximum vers un minimum à une seconde limite radiale de la section transversale du faisceau laser.

7. Appareil de sculpture selon l'une quelconque des revendications précédentes, dans lequel l'intensité de la projection du faisceau laser est limitée à un niveau prédéterminé tel que seule une fraction d'une pénétration maximale prédéterminée dans la région du stroma de la cornée peut être accomplie dans une période de temps prédéterminée, et des moyens (16) sont prévus pour régler le temps d'exposition de la cornée au faisceau précaractérisé afin qu'un seul et unique même moyen précaractérisant puisse être utilisé pour effectuer une modification présélectionnée parmi plusieurs modifications dioptriques différentes prédéterminées.

8. Appareil de sculpture selon l'une quelconque des revendications précédentes, comprenant des moyens (17) pour stabiliser la tête du patient par rapport au faisceau laser.

9. Appareil de sculpture selon l'une quelconque des revendications précédentes, comprenant un dispositif de fixation optique (20) incluant un réticule et un objectif de visée, ce par quoi l'oeil, que l'on n'opère pas, peut voir le réticule comme s'il était à l'infini.
